# EUROPEAN PATENT APPLICATION

(11) **EP 2 133 087 A2**
(43) Date of publication of application: **16.12.2009**
(21) Application number: 09011764.9
(22) Date of filing: 28.09.2004
(51) Int. Cl.: A61K 35/14, A61M 1/34, A61M 1/36

(54) **Cell bank for contingent autologous leukocyte transplantation**

(30) Priority: 30.09.2003 GB 0322806
(62) Divisional of application: 04768677.9
(71) Applicant: Lifeforce Immune System Bank PLC, Imperial House Imperial Way Newport Gwent NP10 8UH (GB)
(72) Inventor: Spiers, William Arthur, North Bradley Wiltshire BA14 9RP (GB)
(74) Representative: Hutchins, Michael Richard

(57) **Abstract**

A process for producing a leukocyte bank suitable for CAT therapy comprising the steps of providing a blood sample from a healthy donor individual selectively separating leukocytes from the sample; collecting the separated leukocytes in a holding vessel; transferring two or more aliquots of the separated leukocytes to independent double containment storage vessels, wherein each of the storage vessels contains, or is in fluid communication with, a cryopreservation medium; mixing each aliquot with the cryopreservation medium within each storage vessel; cryogenically preserving each of the two or more aliquots within each of the storage vessels, retrievably depositing each of the two or more storage vessels containing the aliquots of preserved leukocytes into two or more independent storage systems to produce a leukocyte bank which exhibits deposit redundancy, wherein steps are conducted within a closed or functionally closed system and are applied iteratively to a series of blood samples from different healthy donor individuals. The process further comprises digitally storing information obtained from each donor individual in a digital information unit so as to permit matching of leukocyte deposit and donor for later autologous transplantation.

## Description

### Field of the Invention

The invention relates to a process for producing a leukocyte bank, to leukocyte cell banks and preserved leukocyte compositions created thereby and to various forms of therapy based thereon.

### Background to the Invention

### Cell banks

Cell banking is a service industry in which live cells are stored for later use. It has been practised for decades, and is exemplified by the storage of bovine sperm cells for the artificial insemination of cows.

With the technical advances that are being made in bio-medical research and tissue engineering, it is being recognized that many possibilities may exist for the use of human stem cells for various replacement therapies. These developments have led to a growing demand for facilities where stem cells of individuals can be isolated, cryo-preserved, and stored for later (autologous) use. For example, the desirability of storing the cord blood stem cells of newborns is becoming increasingly recognized and as a result there is a rapidly increasing number of deposits of such stem cells in private cell banks.

With this growth in interest in cell and tissue banking has come an increasing awareness of the practical problems. It has become clear that cell banks intended to provide a long-term cellular resource are vulnerable to random events that lead to loss of viability of some or all of the deposits and that the risks associated with such events increase with the size of the bank and with the duration of storage. Deposit integrity is also crucially important: the way in which the deposits are prepared, stored, handled and used may crucially determine the integrity of the bank; this is particularly important when cross-contamination of deposits can lead to the spread of disease or to inappropriate or dangerous physiological consequences (such as may arise from the administration of allogenous cellular material when autologous grafting is indicated). With large banks, information storage, processing and deposit cataloguing are also extremely important.

Such issues have lead to a growing number of statutory provisions and codes of practice governing the production, maintenance and use of cell banks in most countries: in the United Kingdom, cell banking is now controlled by a comprehensive regulatory framework.

### Contingent autologous transplantation (CAT) therapy

A form of therapy has recently been described (see WO 00/29551 and WO 01/88099) in which various tissues (including leukocytes) are removed from a healthy donor and stored in a tissue or cell bank for later autologous transplantation in the event that a need for such autotransplantation arises at some future date. This form of therapy is herein referred to as *contingent autologous transplantation* (CAT) therapy.

For any given tissue or cell type, the need for CAT therapy is likely to arise in only a fraction of the healthy population. As a result, the effectiveness of CAT therapy depends crucially on the generation of comprehensive cell and tissue banks in which deposits from a large percentage of the population are included.

Accordingly, it has been proposed that CAT therapy be facilitated by the construction of comprehensive tissue banks. However, the nature of CAT therapy places unique and stringent demands on any such tissue bank. In particular, CAT therapy implies a large number of participating donors (and consequently a large number of deposits), relatively long-term storage, good retention of tissue function over time and great flexibility in ultimate therapeutic use.

Such problems are particularly acute in the case of leukocyte cell banks, where the absolute number of cells available is relatively small, the ultimate therapeutic efficacy may depend critically on the function of a small subset of cells and the activity profile of the stored leukocytes may change over time as the various subsets of cells respond to storage in different ways. To date, no leukocyte cell banks suitable for CAT have been constructed.

### Isolation of leukocytes for banking

### (a) Centrifugal leukocyte extraction from whole blood

It is known that whole blood can be readily separated into three discrete components by centrifugation. Centrifugation (e.g. at about 3900 rpm) partitions the various components of the whole blood into three distinct layers: a *blood plasma* layer (of relatively low density), and *red blood cell* layer (of relatively high density) and a layer of intermediate density consisting predominantly of leukocytes and thrombocytes (platelets). This latter layer normally appears at the interface between an upper plasma and lower red blood cell layer after centrifugal separation, and is known as the *buffy coat.*

Such separation is presently carried out, for example on donated blood, in order that plasma, red cells and thrombocytes can be processed separately. This reflects the distinct storage regimens and potential therapeutic applications appropriate to each fraction. For example, the concentrated red blood cell fraction is usually stored chilled for up to 35 days, and can be used for treating anaemic patients, victims of trauma and patients undergoing surgery. The plasma is generally frozen below -30ºC for up to one year, and can be used to reverse anticoagulant treatment and as a component in massive transfusions. The thrombocytes, stored at 20°C and continually agitated to prevent clotting, can be used to prevent bleeding in leukaemic patients or those undergoing chemotherapy or massive blood transfusions.

The leukocytes present in the buffy coat fraction are usually regarded as an unwanted contaminant, since they can induce profound and possibly dangerous immunological changes if transfused inappropriately. For this reason, steps are usually taken to limit the collection of these cells during blood sampling (e.g. by the use of in-line filters during blood donation).

While the centrifugation of whole blood can be done in rigid vessels, it is most efficiently carried out on the flexible bags in which the blood is most conveniently collected. Such bags are usually fitted with flexible tubes opening into the upper and/or lower ends of the bags and are designed to fit into specially adapted centrifuge rotors, where they can withstand the forces necessary to effect separation of the blood *in situ.*

Such bags are known to those skilled in the art as *blood (press) packs,* and a wide range of different types are now commercially available (e.g. from Baxter Healthcare Ltd.).

Once separated in the blood pack, various types of apparatus have been devised for pressing the flexible walls of the blood pack in order to controllably express one or more of the separated blood fractions for further processing.

Such apparatus, known to those skilled in the art as a *blood (pack or bag) press,* usually features one or more vertical press plates between which the bag can be introduced and which act to drive the different layers successively out of the pack when one plate is advanced towards the other. This usually results in the plasma layer being expressed first, followed by the buffy-coat. The red blood cell fraction can then be expressed last, or retained as a residual fraction in the pack.

Alternatively, the bag press can be designed to express the plasma and red blood cell layers from opposite ends of the pack simultaneously, leaving the buffy-coat as a residual fraction.

Examples of such bag presses are described in US 4350585, US 5874208 and US 4663032. A wide variety are commercially available, for example the Optipress® II from Baxter Healthcare Ltd.

### (b) Leukapheresis

Leukapheresis is a specific form of apheresis which involves the selective separation and removal of leucocytes from withdrawn blood, the remainder of the blood then being retransfused into the donor. During leukapheresis, the removed blood is passed through a cell separation device which separates nucleated white blood cells from red blood cells and plasma outside the body. The red blood cells and plasma are returned to the individual, as part of the separation process. The process is continuous with blood being removed and returned almost simultaneously after various extractions have been performed. Leukapheresis therefore makes it possible to remove and return the entire blood volume of the individual several times over and separate out and keep large numbers of white cells without detriment to the individual. The technique therefore relies on the establishment of a vein-to-vein extracorporeal blood circulation and extraction of leukocytes from the recirculating blood.

Leukaphereses are generally automated, and conducted using either continuous or interrupted flow centrifugation or filtration techniques, as described in "Leukapheresis and Granulocyte Transfusions", published by American Association of Blood Banks, Washington DC (1975).

Apparatus for carrying out centrifugation leukapheresis is described in US 3489145 and US 3655123, while that for carrying out filtration leukapheresis is described in US 3802432 and US 3892236. Gravity leukapheresis, in which the forces for both separating and collecting leukocytes are provided by gravity alone, is described in US 4111199.

Many different types of automated leukapheresis apparatus are now commercially available including the Fenwal CS-3000 (Baxter Healthcare, Chicago, IL), the Cobe 2997 (Cobe BCT, Lakewood, CO), the Cobe Spectra, the Cobe 2991, and the Haemonetics V50 (Haemonetics Corp., Braintree, MA). Any of these systems can be used in the processes of the invention, but preferred is the Cobe® system (Cobe BCT, Lakewood, CO, USA), which is capable of extracting between 40% and 50% of the total white cells in the whole blood that passes through the separator, and which can achieve a flow rate of 40-60 ml or more per minute.

Leukapheresis has recently been proposed as a means for creating lymphocyte cell banks (see WO 00/29551 and WO 01/88099).

The present inventors have now recognized that contingent autologous transplantation of leukocytes requires a specific combination of blood processing techniques and cell bank construction in order to meet fulfil the unique demands imposed on a leukocyte cell bank by CAT therapy, which include *inter alia* the need for reliable matching of autologous material, exceptionally robust long-term storage, retention of leukocyte functionality and flexibility in ultimate therapeutic potential.

In particular, the present inventors have found that a combination of: (a) deposit redundancy; (b) stringent control over cryogenic preservation parameters through the use of separate holding and storage vessels; (c) the use of closed (or functionally closed) blood processing apparatus; (d) digital storage of information, and (e) double containment vessels for cell storage is essential for fulfilling the unique demands made on a leukocyte bank intended to support CAT therapy.

### Summary of the Invention

Accordingly, in a first aspect the present invention provides a process for producing a leukocyte bank suitable for CAT therapy comprising the steps of:
(a) providing a blood sample from a healthy donor individual;
(b) selectively separating leukocytes from the sample;
(c) collecting the separated leukocytes in a holding vessel;
(d) transferring two or more aliquots of the separated leukocytes to independent double containment storage vessels, wherein each of the storage vessels contains, or is in fluid communication with, a cryopreservation medium;
(e) mixing each aliquot with the cryopreservation medium within each storage vessel;
(f) cryogenically preserving each of the two or more aliquots within each of the storage vessels;
(g) retrievably depositing each of the two or more storage vessels containing the aliquots of preserved leukocytes into two or more independent storage systems to produce a leukocyte bank which exhibits deposit redundancy;
   wherein:
(h) steps (b) to (g) are conducted within a closed (or functionally closed) system and are applied iteratively to a series of blood samples from different healthy donor individuals; and
(i) the process further comprises digitally storing information obtained from each donor individual in a digital information unit so as to permit matching of leukocyte deposit and donor for later autologous transplantation.

The holding vessel serves to hold the leukocytes during the isolation and collection steps (which may take several minutes or even hours to complete) and permits stringent control over the mixing and preserving steps. In particular, the use of a separate holding vessel permits control over the incubation time and concentration of the leukocytes in the cryogenic preservation medium prior to freezing.

The present inventors have found that this precaution significantly improves the retention of leukocyte functionality after subsequent cryogenic preservation and revitalization and is essential for creating a reliable leukocyte bank in which the viability of a subset of leukocytes in a relatively small number of autologous deposits may ultimately prove to be critical to successful CAT treatment.

The present inventors have also found that the size of a leukocyte bank (in terms of the number of deposits) necessary to serve as the basis for broadly applicable CAT therapy is so large that the chance of proximal barrier failure p, although small at the level of individual deposit, becomes critical to the integrity of the bank as a whole. This is because in cases where the bank size (the number of filled storage vessels n) approaches or exceeds 1/p, proximal barrier failure in any one storage vessel becomes inevitable over the lifetime of the bank. Since the cell bank must serve as a resource for CAT therapy, the use of uncontaminated, autologous leukocytes is critical: such an event could therefore compromise the integrity (and usefulness) of the entire bank.

Accordingly, independent double containment storage vessels are used in the process of the invention. The term *double containment,* as used herein in relation to the storage vessels of the invention, is a term of art defining a container having at least two barriers: a proximal barrier, in contact with the separated leukocytes, which contains the leukocyte sample and isolates it from the outside environment, and a secondary barrier which acts as a fail-safe to isolate the leukocyte sample from the outside environment in circumstances where the proximal barrier fails (for example after accidental physical or chemical trauma).

The term *independent,* as applied to the storage vessels of the invention, is intended to define vessels which can be independently stored (as defined herein). Thus, the independent storage vessels of the invention may have non-contiguous internal volumes, may not share a barrier and may be entirely separate (or separable) vessels.

The storage vessels of the invention contain, or are in fluid communication with, a cryopreservation medium. In preferred embodiments, the cryopreservation medium is simply present in liquid form within the storage vessel. In such embodiments, the cryopreservation medium may be isolated from the holding vessel(s) by any convenient means, for example by: (a) a valve; (b) a breakable seal, (c) gravity in conjunction with the spatial arrangement of the holding and storage vessels; and/or (d) surface tension within a tube connecting the holding and storage vessels.

In other embodiments, the cryopreservation medium is in fluid communication with the storage vessel but not contained therein, for example being held in a satellite cryogenic medium vessel. In such embodiments, the satellite cryogenic medium vessel may be reversibly isolated from the storage vessel by any convenient means, for example by (a) a valve; (b) a breakable seal, (c) gravity in conjunction with the spatial arrangement of the satellite and storage vessels; and/or (d) surface tension within a tube connecting the satellite and storage vessels.

As a further precaution, two or more aliquots are used according to the invention in order to provide for deposit redundancy. Preferably, three, four, five or greater than five separate aliquots are used according to the invention.

The storage vessels are preferably detachable from the holding vessel and from any ancillary tubing and/or satellite vessels, for example by releasable clamps, releasable docking components, tearable seals, heat crimping or by use of so-called "docking systems" for sterile sealing.

The independent storage systems into which the aliquots are retrievably deposited are independent in the sense that they do not share a determinant of viability selected from: (a) power supply and/or (b) site or location. For example, in cases where the storage systems depend on a supply of electricity for their continued cryopreservation, then the electricity supplies must not originate from a single generator or supplier.

Preferably, each independent storage system is sited to be geographically remote from its counterpart(s), so lessening the chances of coincidental destruction or damage by natural or man-made disasters (such as fire, flood or contamination).

As used herein, the term *closed system,* in the context of the process of the invention, is used to define blood processing apparatus consisting of elements which are sterile and isolated from the outside environment by aseptic barrier(s) and in which all components are fully integral, being attached and/or assembled at the manufacturing site.

As used herein the term *functionally closed system,* in the context of the process of the invention, is used to define blood processing apparatus consisting of elements (e.g. tubing sets) which are assembled at the device manufacturing site and which use sterile barrier filters (e.g. 0.22 micron filters) or so-called "docking systems" for the sterile interconnection by the end user to generate a wide variety of arrays of tubing, channels, filters, satellite bags and other vessels.

The cryogenic preservation step (f) conveniently comprises freezing to a temperature at or below about -160°C, which can be achieved using liquid nitrogen. If longer periods of storage and/or enhanced retention of functionality are required then freezing to a temperature at or below about -269°C may be effected using liquid helium.

Any of a wide range of suitable cryopreservation media may be used according to the invention, but preferred are media comprising a suitable penetrating cryoprotectant. Particularly suitable for use as a penetrating cryoprotectant is DMSO, which may be used for example at a concentration of up to 10%.

The cryopreservation medium may further comprise an anticoagulant (such as acid citrate dextrose, EDTA, heparin or mixtures thereof), a nuclease (for example a Dnase and/or Rnase as well as a physiologically acceptable medium (for example, phosphate buffered saline). The cryopreservation medium may also further comprise a proteinaceous composition, such as blood serum or a blood serum component and/or a sugar and/or a polysaccharide (which may be particularly preferred in embodiments where plunge freezing is employed).

Particularly preferred proteinaceous compositions for use in the cryogenic preservation media of the invention comprise blood albumin (e.g. bovine serum albumin or human serum albumin). Particularly convenient is the use of human blood serum isolated from the blood sample of the donor individual. This can be isolated as a coproduct together with the leukocytes.

Any donor may be used as a source of blood sample in the processes of the invention, provided that the donor is healthy, as herein defined. However, the invention finds particular application in relation to donor individuals which are predisposed to a leukocyte deficiency, are not in remission from a leukocyte deficiency, are juvenile, adolescent or adult, are at risk of developing a leukocyte deficiency, are human individuals between the ages of about 12 to 30 (e.g. 15 to 25) and/or have a fully-developed immune system.

The blood sample for use in the process of the invention may be one which is in fluid communication with the donor individual (for example, in circumstances where leukapheresis is used to selectively separate the leukocytes). Preferably, however, the sample is an isolated blood sample (as defined herein).

The leukocytes separated in step (b) may comprise (or consist essentially of) granulocytes, lymphocytes and/or monocytes. In many embodiments of the invention, no steps are taken to enrich for (or isolate) any particular class (or classes), subpopulations or cell types.

However, in some embodiments the separation step (b) comprises the selective separation of a particular class or type of leukocyte. This may be done in circumstances where it is desired to effect the selective separation of B-cells and/or T-cells and/or dendritic cells (or mixtures thereof).

The information stored in step (i) comprises at least that necessary to permit matching of deposit with donor, in order that later autologous transplantation can be carried out. Preferably, the information comprises genetic information, the date at which the blood sample was collected from the donor individual, the age and sex of the donor individual, the clinical status of the donor individual, the medical history of the donor individual, biographical data identifying the donor individual, details of the processing and storage conditions used to prepare the deposit as well as data identifying the person(s) responsible for processing the sample(s).

If genetic information is stored, then this preferably comprises sequence information relating to one or more gene(s), single nucleotide polymorphism (SNP) data and/or one or more genetic fingerprint(s).

Any suitable digital information unit may be used to store the information. Preferably, this takes the form of at least one digital computer comprising a database. The database may carry data on a carrier of any convenient form. Preferably, the information is stored independently on two or more carriers so that the database exhibits redundancy. This protects against data loss in the event of failure, corruption or loss of one of the computers or data carriers.

Preferably, the process further comprises the step of labelling the storage vessels of step (f) with information sufficient to permit matching of the leukocyte deposit and donor. For example, the storage vessels may be labelled with information:
(a) describing the contents of the vessel (for example, sample size, number and/or volume); and/or
(b) identifying the leukocyte bank; and/or
(c) recording the date at which the blood sample was collected from the donor individual; and/or
(d) comprising a statement that each package is for single patient use only; and/or
(e) comprising instructions for opening, aseptic presentation and further storage.

Any convenient form of labelling may be used. Thus, the labelling may comprise the physical attachment of an information carrier (e.g. a bar code) to the storage vessels themselves. Alternatively, the labelling may be effected by the non-physical association of the vessels with the information carrier (for example, via the correlation between the physical geometry or organization of the deposits in the bank and the entries in the database).

The invention also contemplates treatment of the leukocytes, for example including any or all of the following: *in vivo* prior to provision of the blood sample, *in vitro* prior to separation step (b), *in vitro* after separation step (b) but prior to preservation step (f) and/or *in vitro* after preservation step (f).

Any suitable method for selectively separating the leukocytes from the sample may be used. In preferred embodiments, the leukocytes are selectively separated in step (b) using leukapheresis apparatus (as described in more detail herein).

The leukapheresis apparatus preferably comprises: (a) a separation device (e.g. a centrifuge rotor or filter); (b) a leukapheresis tubing set; and (c) one or more pumps for conveying the sample through the tubing set and the separated leukocytes into the collection vessels.

Particularly preferred is the separation and collection of leukocytes in steps (b) and (c) using automated leukapheresis apparatus.

Particularly preferred is the use of automated leukapheresis equipment in isolated leukapheresis mode (as defined herein), or in the more usual mode of automated continuous or interrupted flow centrifugation leukapheresis or continuous or interrupted flow filtration leukapheresis.

Non-automated or manual techniques may also be used to separate and/or collect the leukocytes in steps (b) and (c) of the process of the invention. For example, batch centrifugal separation and collection (e.g. by bag pressing) and gravity leukapheresis (as described in US 4111199) may be employed, provided that the blood processing elements (blood packs, tubing, satellite vessels etc.) together define a closed (or functionally closed) system.

In another aspect, the invention contemplates a process for producing a leukocyte composition for autotransplantation into a donor individual comprising the steps of:
(a) producing a leukocyte cell bank by the process of the invention;
(b) matching the donor individual with a leukocyte deposit to identify an autologous leukocyte deposit using the information stored in step (h);
(c) retrieving a storage vessel containing an aliquot of preserved autologous leukocytes;
(d) revitalizing the preserved autologous leukocytes to produce a leukocyte composition for autotransplantation into the donor individual.

The invention also contemplates a leukocyte composition and a leukocyte cell bank obtainable (or obtained) by the process of the invention.

Also contemplated are various therapeutic uses for the processes, compositions and banks of the invention. Accordingly, the invention contemplates the leukocyte composition of the invention for use in therapy, for example in CAT therapy.

### Detailed Description of the Invention

### I. Definitions

Where used herein and unless specifically indicated otherwise, the following terms are intended to have the following meanings in addition to any broader (or narrower) meanings the terms might enjoy in the art:

The term *leukapheresis* is a term of art used herein to define a procedure involving the selective separation and removal of leukocytes from the withdrawn blood of a donor, the remainder of the blood then being retransfused into the donor.

A *leukapheresis device* is a term of art defining any device capable of performing leukapheresis, irrespective of the means employed in the device to separate and remove the leukocytes.

The term *isolated leukapheresis* is used herein to define a novel form of leukapheresis which is performed on an *isolated* blood sample.

The term *isolated blood sample* is used herein to define a blood sample which is not in fluid communication with the blood of the donor from which it originated. Thus, in the process of isolated leukapheresis which is applied to isolated blood samples, the leukapheresis device is not in fluid communication with the individual providing the blood sample and/or the remainder of the blood in the sample is not retransfused into the individual.

The term *autotransplantation* is used herein to define autologous transplantation (autogeneic or self-to-self transplantation), wherein the term *autologous* is used to indicate that the transplantation is to the same organism (i.e, the same individual) from which the cellular material (e.g. leukocytes) was removed. As used herein, *transplantation* defines any procedure involving the introduction of cellular material (e.g. leukocytes) into an organism, and so any form of transplantation or grafting known in the art is encompassed.

The term *dormancy* is used herein to define any state of suspended animation or stasis, and procedures for achieving this are well known in the art, as described below. Any of the known procedures may be used, including cryopreservation. Thus, the leukocytes may be held or maintained in a quiescent, inactive or non-proliferating state.

The term *healthy* is used herein in relation to an individual donor to indicate that the individual is not suffering from a leukocytic deficiency (as herein defined). Thus, the term *healthy* as used herein encompasses *non-diseased* individual donors in a state in which the individual donor is not suffering from any disease or disorder, or is not manifesting any symptoms of said disease or disorder (i.e. is asymptomatic or is in a pre-clinical condition). In particular, term *healthy* as used herein encompasses individual donors not suffering from, or demonstrating symptoms of, the disease or disorder which it is subsequently intended to treat by the autotransplantation procedure.

### II. Blood samples

The invention may be applied to any form of blood sample, provided that the sample comprises at least some leukocytes from the individual donor. The blood sample may be subjected to various treatments *ex vivo* prior to use in the process of the invention. Typically, for example, the blood sample is chilled prior to use. Other treatments may include the addition of preservatives and/or anticoagulants.

The blood sample may also be treated *in vivo* prior to collection by administering various agents to the donor individual before or during sample collection. Examples of treatments (which may be applied *ex vivo* and/or *in vivo*) are discussed in more detail in the section entitled "Leukocyte treatments", below.

It is generally preferable to sample at least 450-500 ml of blood from the individual donor, which is the equivalent of a unit of blood as provided by a blood donor for the UK blood transfusion service. If possible a number of samples (e.g. several 450-500 ml samples) are taken over a period of time (e.g. over 2-3 weeks, preferably 2-3 months or over 6 months or a year, 2 or 3 years or more). One or more of these can then be divided or combined into a number of leukocyte cell bank deposits. The removal of a unit of blood is commonplace with over three million units of blood being taken, for allografting, from individuals annually in the UK alone.

The blood removed is soon replaced and, therefore, multiple samplings of a unit of blood from an individual can be provided over a year, say 2-12 unit samplings if necessary, without detriment to the individual being sampled.

### III. Selection of donor individuals

### General considerations

Restorative autotransplantation is a form of therapy that might ultimately be indicated for any individual. Consequently, the invention may be usefully applied to the generation of comprehensive leukocyte cell banks covering as large a number of different individuals as possible in order that restorative autotransplantation can be carried out in any of the represented individuals should the need arise.

It is therefore contemplated that the invention be applied as broadly as possible so that a comprehensive leukocyte cell bank can be assembled. However, since the quality of the individual deposits will depend (at least to some extent) on the health status of the individual donor at the time of blood sample donation, it is preferred that the blood sample for use in the processes of the invention be taken from healthy individual donors.

Other factors also affect donor selection: for example, the blood sample for use in the processes of the invention may advantageously be obtained from individual donors when they are young, preferably in adolescence or early adulthood. In the case of humans, blood sampling (preferably multiple sampling) at the ages of about 12 to 30, preferably 15 to 25 is preferred. Especially preferably, sampling is from the age of 16 or 17 upwards, for example in the age range 16 to 30, 17 to 30, or 18 to 30, or perhaps 18 to 35 or 40. It is thus preferred that the cells be obtained when the host organism is mature, or reaching maturity, but before the processes of ageing or senescence have significantly set in. In particular, it is preferred and advantageous that the immune system of the host organism is mature or fully developed.

However, the obtention of cells outside these ranges is encompassed, and cells may be obtained at any post-natal life stage e.g. from juvenile host organisms e.g. in mid-to late childhood, or even infants, or from older individuals.

Sampling from post-natal or older hosts allows multiple samples to be Collected, thereby increasing the opportunity of storing sufficient number of cells. In addition sampling from juvenile or older hosts overcomes the ethical requirements such as providing informed consent.

Sampling from adolescent or adult host organisms is preferred since the sampled cells, from blood in particular, will contain a greater proportion of valuable mature T-cells capable of recognising aberrant cell populations, such as cancer cells or virally infected cells. Thus, when blood samples are used, it is advantageous that they are taken from an individual with a mature immune system (i.e. not foetal or neonatal).

Thus, the invention contemplates the use of blood samples collected from donor individuals at a stage when there is no direct prediction, suggestion, or suspicion that a particular disorder or disease may develop, for use against a future possible or unpredicted event, or an event which may occur simply by chance, rather than an anticipated or suspected or predicted illness or condition. Thus, in certain embodiments of the invention, the donor individual is not predisposed to, or at risk from, any particular disease or disorder e.g. not exhibiting any symptoms or manifestations predictive of a subsequent disease or disorder. Likewise, the host organism is preferably not suffering from any injuries or damage which may give rise to an anticipated or expected condition.

Indeed, for certain applications (for example, the generation of leukocyte cell banks for subsequent restorative autotransplantation) it is preferred that the blood sample for use in the invention be obtained from the donor individual before any disease or disorder develops or manifests itself, and more preferably when the host organism is in general good health, and preferably not immunocompromised in any way. In such embodiments it is particularly advantageous to sample the blood from donor individuals at a time when the organism has not previously exhibited symptoms of or presented with or been diagnosed as suffering from the disease or disorder which is subsequently to be treated, i,e, when the host organism is healthy and not "in remission" e.g. not in a state of partial or full recovery from the leukocyte deficiency to be treated.

### Predisposed donor individuals

Advances in therapy continue to be made, and our greater understanding of disease processes helps us to modify and refocus our therapeutic approaches to alleviate disease and suffering. Such understanding has been greatly advanced by technological improvements in the field of molecular biology. We are now in a position to follow the pathogenesis of diseases at a molecular level, and recognize the importance of an individual's genetic make-up in predisposing them to certain diseases. For example, we are aware that some individuals, because of their genetic composition, are prone to certain diseases.

Many of the diseases to which certain individuals can be predisposed are leukocyte deficiencies, which term is used herein to indicate a condition in which the administration of autologous leukocytes is indicated. Such conditions therefore include those in which an individual has acquired a disease, infection or condition involving leukocyte dysfunction or a disease, infection or condition in which the augmentation or stimulation of endogenous leukocyte activity is indicated. Detailed examples of particular leukocyte deficiencies are set out in the section entitled "Exemplary indications", below.

Through genetic testing, therefore, it is now possible to identify those individuals predisposed to a leukocyte deficiency (e.g. any of various forms of cancer, immune disorder or infection).

Furthermore, our knowledge of the body's immune system, and in particular the way in which it recognises and kills virally infected and tumour cells, continues to advance. We now know that in order to elicit cell-mediated immunity, an offending cell (e. g. a virally infected or tumour cell) must co-present an HLA class I restricted tumour or viral epitope with danger signals such as GM-CSF and/or TNF-alpha, so that the antigen presenting cells (APC) of the immune system will express co-stimulatory signals such as B7 and IL-12 in conjunction with antigen to the interacting cytotoxic T-lymphocyte (CTL) population. The co-presentation leads to the production of clones of both activated and memory cells (for review see Nature Medicine Vaccine Supplement 4 (1998) 525). In the absence of these additional signals, HLA-I antigen-restricted T-cells which recognise offending cells are processed for destruction or desensitization (a bodily process presumably put into place to avoid the development of e.g. autoimmune disease). The induction of such tolerance is because of either ignorance, anergy or physical deletion (Cold Spring Harbour Symp Quant Biol 2 (1989) 807; Nature 342 (1989) 564; Cell 65 (1991) 305; Nature Med 4 (1998) 525).

It is now clear that tumour cells do not automatically co-present danger and/or co-stimulatory signals. Hence, the spawning of a tumour may lead to eradication of the very T cell clones that provide cell-mediated immunity against the tumour. A patient presenting with a cancer, leukaemia/lymphoma or sarcoma etc, therefore, may have already removed their innate ability to destroy the tumour, by default.

However, if the required T lymphocytes, or a sample thereof, were removed from the patient prior to the onset of proliferative disease, the relevant T-cell population could now be returned to the patient, after the necessary co-stimulation of the T-cells, so as to alleviate disease. Co-stimulation may be provided at the same time as the cells are returned to the patient, or after they are returned through further treatment (s) of the patient, or without stimulation other than that naturally produced by the patient. Activation/stimulation of the cells may also initially be induced *in vitro* prior to reinfusion.

The present invention therefore finds particular application in the case of individuals predisposed to the development of a leukocyte deficiency. It therefore represents a means for removing leukocytes from a healthy donor individual for subsequent transplantation to that same individual in a subsequent autologous (autogeneic) transplantation procedure, when the need or desire to do so arises. Although the predisposed individual may never receive the cells because no disease to be treated by this method ever occurs, the invention nevertheless may be used to provide some form of insurance against the heightened risk of a leukocyte deficiency arising in the individual.

Similarly, individuals with no diagnosed predisposition may choose to provide samples for incorporation into the leukocyte cell bank of the invention for prospective use by themselves prior to travelling abroad. Such use might include for the treatment of infections contracted whilst abroad.

In addition, it is well recognized that the ageing process makes individuals more susceptible to disease. The basis for the susceptibility appears to be in the loss of immune function resulting from a significant decrease in T and B cell numbers/activity during ageing (Mech Ageing & Dev 91 (1996) 219; Science 273 (1996) 70; Mech Ageing & Dev 96 (1997) 1). Disease susceptibility is particularly pertinent when elderly patients are subjected to e.g. surgery in a hospital environment, where they are prone to opportunistic infections with serious or even fatal consequences. Blood samples taken from such individuals much earlier in life and processed according to the invention for inclusion in a leukocyte cell bank could provide the opportunity for restorative autotransplantation in such circumstances.

Such an approach could be used more broadly to provide for a method of augmenting the patient's immune system after surgery in order to lessen the likelihood of post-operative complications caused by opportunistic infections. The invention, therefore, could be used as a prophylactic therapy, e.g. for elderly patients when they are more susceptible to disease.

### IV. Leukocytes

It will be appreciated that the separation and/or removal of leukocytes from the blood sample during leukapheresis need not be absolute. Rather, the removal and/or separation of a fraction of the total leukocytes present in the sample is sufficient in most circumstances. Those skilled in the art will readily be able to determine the appropriate size of the fraction to be removed, which will vary *inter alia* according to the use to which the isolated leukocytes are to be put, the size of the sample, the status of the donor, the nature of the leukocytes and the particular leukapheresis device employed.

The leukocytes collected in the processes of the invention are to some degree isolated from the original blood sample. The term *isolated* is used here to indicate that the isolated leukocytes exist in a physical milieu distinct from that in which they occur *in vivo* and does not imply any particular degree of purity. Indeed, the absolute level of purity is not critical, and those skilled in the art can readily determine appropriate levels of purity according to the use to which the leukocytes are to be put.

The separation and collection of the leukocytes in the processes of the invention also does not necessarily imply that any particular class or type of leukocyte is preferentially separated and collected. Rather, the leukocytes of the invention include any white blood cell, including granulocytes, lymphocytes and monocytes.

Granulocytes include myelocytes, basophils, eosinophils and neutrophils. Lymphocytes include B, T lymphocytes and natural killer cells. Monocytes include mononuclear phagocytes and other macrophages.

However, in some embodiments the leukocytes which are separated and collected preferably comprise one or more specific leukocyte cell types. A preferred cell type is the lymphocyte, especially a T-lymphocyte (T-cell). Mature T-lymphocytes are particularly preferred.

Since the total mature T-cell number per litre of blood ranges between 1-2.5 x 10⁹ for humans, a 100 ml sample of blood typically contains 1-2.5 x 10⁸ mature T-cells and this is generally sufficient to provide an adequate representation of the entire mature human T-cell population for the beneficial effect. However, depending on the fraction of total leukocytes separated and collected by the leukapheresis device and the efficiency of any revitalizing technique employed, preferably at least 100 ml, 115 ml, 200 ml or 300 ml and even more preferably in excess of 400 or 500 ml of blood sample is used in order to obtain the appropriate number of mature T-cells to support a beneficial therapeutic effect for return to the individual if and when they become ill.

Standard techniques are known in the art which permit selection of particular subpopulations of lymphocytes from a sample comprising a mixed population of lymphocytes. Examples of such subpopulations are CD3⁺, CD8⁺, CD4⁺ and CD16/56⁺ (natural killer) T cells and CD19⁺ B cells. For example, any one or any mixture or combination of such subpopulations of T cells can be used in the methods, uses and compositions of the invention, and they are readily obtained by means of well known methods such as FACS (Fluorescence Activated Cell Sorting) and haemocytometry systems.

### V. Leukocyte treatments

The leukocytes may be subjected to various treatments. Such treatments may, for example, result in expansion of some or all of the representative cell subsets, improve the long-term viability of the leukocytes during the dormancy period, improve their therapeutic potency and/or render their subsequent use in autotransplantation safer.

The treatments can be carried out before or after the leukocytes are rendered dormant (and before or after autotransplantation is carried out). Moreover, the treatments may be applied after the blood sample is taken (i.e. be carried out *ex vivo*) either prior to rendering the cells dormant or after revitalization. For example, treatment of the leukocytes may be effected by co administration of a separate composition, sequentially or simultaneously with the leukocyte composition, during autotransplantation. Treatment of the leukocytes can be effected immediately prior to autotransplantation. Alternatively (or in addition) the treatments may be applied to the leukocytes while still *in vivo* prior to blood sampling by the administration of e.g. growth factors or cytokines (see below).

Exemplary pre-transplantation treatments may include various genetic modifications, such as the incorporation of a negative selection marker (as described, for example, in WO96/14401, the content of which is incorporated herein by reference). Such treatment permits ablation of the leukocytes after transplantation or titration of dose versus response. Other genetic interventions may include regulating or modifying the expression of one or more genes (e. g. increasing or decreasing gene expression), inactivating one or more genes, gene replacement and/or the expression of one or more heterologous genes). Other genetic modifications include the targeting of particular T-cells (as described in WO96/15238, the content of which is incorporated herein by reference), and the modification of the T-cell receptor repertoire/expression with antibodies to make T-cell chimaeras.

Other treatments contemplated by the invention include the exposure of the leukocytes with one or more stimulatory molecules, for example antigens (e.g. cancer or viral antigens), antibodies, T cell recognition epitopes, peptides, blood factors, hormones, growth factors or cytokines or combinations thereof.

For example, the leukocytes may be treated *in vitro* (or *in vivo* prior to blood sampling) with antigens (for example cancer (e.g. prostate-specific antigen 1 or prostate-specific antigen 2, her-2/new, MAGE-1, p53, Haras and c-myc) or viral antigens), antibodies, T cell recognition epitopes, peptides, blood factors, hormones, growth factors or cytokines or combinations thereof. The stimulatory molecules may be synthetic, recombinant or may be purified or isolated from the human or animal body. Particularly useful in this respect are stimulatory molecules selected from IFN-alpha, IFN-beta, IFN-gamma, II-1a, II-Ib, II-2, II-3, II-4, II-5, II-6, II-7, II-8, II-9, II-10, II-11, II-12. II-13, II-14, II-15, GM-CSF, M-CSF, G-CSF, LT and combinations of two or more of the foregoing. Such treatments may modify the growth and/or activity and/or state of differentiation of the leukocytes, and/or may serve to separate or selectively isolate or enrich desired leukocyte cell types or to purge unwanted cells.

Recent advances have been made in the way cells may be obtained for subsequent autotransplantation. For example, investigations into the agents which regulate haematopoiesis have led to the isolation of a series of factors that influence the proliferation and differentiation of lymphocytes. These agents include the cytokines (such as the interleukin series IL-1 to IL-18 and the leukotrienes) and growth factors such as the TNF's, the TGF's, FGF's, EGF's, GM-CSF, G-CSF and others. A number of these factors are now available commercially for clinical use, and some have been shown to increase substantially the number of lymphocytic cells and, in particular, immature T-lymphocytes in the peripheral blood. Their administration to the donor individual prior to blood sampling permits the quantity and/or quality (in terms of the number and nature of leukocyte subtypes present) to be controlled and makes it possible to recover large quantities of the cells of interest, e.g. immature T-lymphocytes, directly from the donor individuals peripheral blood sample without the need to sample the marrow.

Other pre-transplantation treatments include culture of the leukocytes (or a sub-population thereof). For example, the leukocytes may be cultured to increase cell numbers. For example, the cells may be passaged, according to methods well known in the art. Such culturing may be carried out before or after the leukocytes are rendered dormant, or both before and after dormancy is induced.

Thus, in the case where the leukocytes include T-cells, the T-cells may be co-stimulated prior to transplantation and/or exposed to tumour antigens (optionally together with co-stimulatory factors) prior to autotransplantation.

### VI. Leukapheresis devices

Many different types of automated leukapheresis devices are presently commercially available. Such devices usually comprise at least three separate elements: (1) a *separation device* (e.g. comprising a membrane or centrifuge rotor, which provides the forces for separating the leukocytes from the various other blood components; (2) one or more *pumps* for conveying the blood sample to the separation device, for removing the separated leukocytes and for maintaining the forces necessary for transfusion and retransfusion, and (3) a (normally disposable) *tubing* set which holds the blood and its various fractions in a particular geometry within the separation device, defines fixed channels through which the blood flows (normally in a circuit from the donor, through the leukapheresis device and back to the donor) as well as vessels (usually bags) for the collection of the separated leukocytes and/or other blood fractions or fluids.

Any of a wide variety of commercially available leukapheresis devices may be used according to the present invention. The particular way in which the leukapheresis device is operated will depend on a number of factors, including the nature of the separation device (e.g. centrifuge, filter etc.), the type of leukocyte sample required, the volume of the blood sample to be processed, the identity and status of the donor individual, the ultimate use to which the leukocyte composition is to be put and the nature of any treatments applied to the blood sample prior to processing according to the invention. Thus, those skilled in the art will readily be able to establish the appropriate operational parameters.

Preferably, however, an automated leukapheresis device is selected to minimize the need for operator intervention and/or training. Commercially available leukapheresis systems vary in the time and/or expertise required of an individual to prepare and operate it. For instance, reducing the time required by the operator to load and unload the tube set, as well as the complexity of these actions, can increase productivity and/or reduce the potential for operator error. Moreover, reducing the dependency of the system on the operator may lead to reductions in operator errors and/or to reductions in the credentials desired/required for the operators of these systems.

Performance-related factors are also relevant, and may be judged *inter alia* in terms of the "collection efficiency" of the leukapheresis system. The "collection efficiency" of a system may of course be gauged in a variety of ways, such as by the size of the fraction of leukocytes collected in relation to the total leukocytes present in the sample. Performance may also be evaluated based upon the effect which the leukapheresis procedure has on the various blood component types. For instance, it is desirable to minimize the adverse effects on at least the leukocytes of the apheresis procedure. It may also be desirable to reduce platelet activation, in order to avoid degeneration in sample quality during processing.

Particularly preferred is the Cobe® system (Cobe BCT, Lakewood, CO, USA).

### VII, Cryogenic preservation

Any suitable means may be employed for cryogenic preservation.

According to a preferred cryopreservation procedure, the cells are frozen preferably to a temperature below-160°C. A particularly preferred means of achieving dormancy is to freeze the cells to the boiling point of helium (He), i.e. to about -269°C or below.

As described in Freshney's (Freshney's Tissue Culture of Animal Cells (Culture of Animal Cells: A Manual of Basic Technique, Wiley Liss, 1994)), the cells may be suspended in a suitable medium (e. g. containing up to 10% DMSO) and cooled at a controlled rate (e. g, 1°C per minute to -70°C, then into liquid/gas N₂). Such conventional procedures may be adapted to cool the cells into He/N₂ mixtures or He. Alternative methods of achieving and/or maintaining cell dormancy include cooling to 4°C.

### VIII. Revitalization

Following dormancy, the cells are revitalised prior to use in transplantation. Again, this may be achieved in any convenient manner known in the art, and any method of revitalising or reviving the cells may be used.

Conveniently, this may, for example, be achieved by thawing and/or diluting the cells. Techniques for revitalisation are well known in the art. Cells may be thawed by gentle agitation of the container holding the cells in water at 37°C, followed by dilution of DMSO to 1% or below, e. g. with medium or serum.

Cells may be implanted immediately or after recovery in culture. Revitalisation is designed to re-establish the usefulness of the cells e.g. in prophylaxis or curative therapy.

### IX. Cell banking

The leukocyte compositions of the invention are banked, thereby creating a leukocyte bank. Any suitable cell banking system may be employed, provided that the deposits are retrievable for autotransplantation. This implies the use of some form of labelling, but this need not be in the form of a physical appendage to the individual deposits.

Thus, the leukocyte cell bank of the invention may comprise a plurality of cell storage units for storage of leukocyte compositions. The cell banks of the invention may further include a digital information unit for digitally storing information relating to the identity, location and medical history of the donor individual and/or the conditions associated with the particular deposit (for example relating to the date at which the blood sample was collected from the donor individual, the processing conditions and details of any treatments applied to the leucocytes contained in the deposit).

The digital information unit preferably comprises at least one digital computer having sufficient digital storage capacity for storage of the potentially large amounts of information relating to each deposit.

The leukocyte cell bank of the invention preferably further comprises an arrangement for digital data retrieval interfaced with the digital information unit for retrieving selected information stored in the digital information unit. The data retrieval arrangement may be integrated with the digital computer. Remote access of the digital information via the telephone or the internet may also be provided and may permit rapid and convenient access of the information on a global basis.

### X. Medical applications

The invention finds application in all forms of therapy and prophylaxis in which the administration of (treated or untreated) autologous leukocytes is indicated (i.e. desirable from a therapeutic perspective). For the purposes of the present invention, in such indications a *leukocyte deficiency* is deemed to have arisen.

It will therefore be understood that the leukocyte deficiencies in which the invention finds medical application encompass a very broad spectrum of diseases, syndromes, disorders, conditions and infections. For example, it will be appreciated that a leukocyte deficiency, in the special, broad sense defined above, can arise in circumstances where an individual has acquired a disease, syndrome, disorder, condition or infection involving leukocyte dysfunction as well as in circumstances where an individual has acquired a disease, syndrome, disorder, condition or infection in which the endogenous leukocyte component is seemingly normal but in which alteration, augmentation or stimulation of the normal endogenous leukocyte activity is nevertheless indicated/required. In particular, a leukocyte deficiency as herein defined may be deemed to have arisen either as a result of a non-specific loss of T- and or B-cells, or as a result of a loss or deficiency of a particular T- and/or B-cell clonal population. For convenience, such diseases, syndromes, disorders, conditions or infections are collectively defined herein as *leukocytic deficiencies.*

The processes of the invention are employed to create a leukocyte composition (e.g. forming part of a leukocyte cell bank) from a blood sample from a healthy individual donor. Thus, the invention is used to create a cellular resource of healthy leukocytic tissue from an individual donor that can be restored to that donor individual should the individual acquire a leukocytic deficiency at a later date.

In such therapies (referred to herein as *restorative autotransplantation*), the invention exploits the fact that many leukocytic deficiencies occur as part of a temporal sequence of events (which may or may not be causally interrelated), so that the creation of a leukocyte cell bank at a point in time predating onset of the leukocytic deficiency constitutes a therapeutic resource which can later be used restoratively.

The concept of restorative autotransplantation described above can be applied to all healthy individuals, irrespective of factors that might serve as indicators of susceptibility to leukocytic deficiency (for example age, medical history, genetic background and lifestyle). However, it does permit the identification of a particular class of individuals for which the processes of the invention may be particularly advantageously applied, as described in more detail in section III (entitled "Selection of donor individuals"), Moreover, since the leukocyte deficiencies as defined above and treatable according to the invention by restorative autotransplantation embrace an enormous variety of known diseases, these are discussed in greater detail in the following section XII (entitled "Exemplary indications").

### XI. Exemplary indications

As mentioned in the preceding section, the therapeutic and prophylactic uses of the invention encompass a very broad spectrum of diseases, syndromes, disorders, conditions and infections.

### Infections

The invention may find application in the treatment of various infections. In this case, the endogenous leukocyte activity may be normal (or responding normally) but its alteration, augmentation or stimulation is nevertheless desirable. In others (such as HIV infection) the endogenous leukocyte activity is dysfunctional as a direct consequence of infection.

Infections which may be treated or prevented according to the invention include bacterial, fungal or viral infections, or infections by any other organism e.g. a protozoan, nematode, insect or other parasite.

A preferred application is the treatment of AIDS as a result of HIV infection. Here, CD4⁺ cells can be collected from an individual when healthy or non-infected, and stored for subsequent transplantation into said individual when HIV infection manifests itself or when AIDS develops, or CD4⁺ cell count falls etc. Such a procedure may be attractive to an individual with a life-style likely to place them at risk from contracting HIV infection.

### Cancers, leukaemias and sarcomas

The invention may find application in the treatment and prophylaxis of various malignancies; in general, any malignant or pre-malignant condition, proliferative or hyper-proliferative condition or any disease arising or deriving from or associated with a functional or other disturbance or abnormality in the cells or tissues of the body.

Therapy or prophylaxis of various forms of cancer represents a preferred embodiment of the invention, and the treatment or prophylaxis of any cancerous cells or tissues of the body is contemplated.

Thus, the invention is not limited to any one type of proliferative disease (e. g. leukaemias, lymphomas, carcinomas or sarcomas), nor is it restricted to specific oncogenes or tumour-suppressor gene epitopes such as prosiate-specific antigen 1 or prostate-specific antigen 2, her-2/new, ras, myc, myb, fos, fas, retinoblastoma, p53 etc. or other tumour cell marker epitopes that are presented in an HLA class I antigen restricted fashion or other such way so as to be identifiable by a leukocyte. All cancers such as leukaemia, lymphoma, breast, stomach, colon, rectal, lung, liver, uterine, testicular, ovarian, prostate and brain tumours such as gliomas, astrocytomas and neuroblastomas, sarcomas such as rhabdomyosarcomas and fibrosarcomas are included for the therapy or prophylaxis by the present invention.

Thus, the present invention finds application in the treatment or prophylaxis of breast cancer, colon cancer, lung cancer and prostate cancer. It also finds application in the treatment or prophylaxis of cancers of the blood and lymphatic systems (including Hodgkin's Disease, leukemias, lymphomas, multiple myeloma, and Waldenstrom's disease), skin cancers (including malignant melanoma), cancers of the digestive tract (including head and neck cancers, oesophageal cancer, stomach cancer, cancer of the pancreas, liver cancer, colon and rectal cancer, anal cancer), cancers of the genital and urinary systems (including kidney cancer, bladder cancer, testis cancer, prostate cancer), cancers in women (including breast cancer, ovarian cancer, gynecological cancers and choriocarcinoma) as well as in brain, bone carcinoid, nasopharyngeal, retroperitoneal, thyroid and soft tissue tumours. It also finds application in the treatment or prophylaxis of cancers of unknown primary site.

### XII. Posology

Those skilled in the art will be readily able to determine the amount of leukocyte composition to be autotransplanted in the medical applications according to the invention. It should be noted that as few as 0.01 x 10⁸ (e.g. 1-10 x 10⁸) mature lymphocytes (which can be derived from a single sample of approximately 100 ml of normal human blood) are sufficient to boost the immune system of a subject and hence may have a beneficial effect according to the autologous transplantation method of the invention. It should be noted that the removal of a unit of blood is commonplace with over three million units of blood being taken, for allografting, from individuals annually in the UK alone.

The leukocyte composition administered may be derived from a single blood sample, or may constitute a pool of leukocyte compositions derived from a plurality of different blood samples taken from a donor individual at different times. The leukocyte composition administered may constitute all or a fraction of the deposited material, but preferably constitutes only a fraction thereof in order that multiple dosing can be achieved, optionally following cellular expansion of the residue (for example, T cell numbers may be increased by *in vitro* expansion using standard methods).

In applications based on T-cell activity, the number of mature T-cells administered is at least 0.01 x 10⁸, more preferably at least 0.1 x 10⁸, more preferably at least 1 x 10⁸ (e.g. at least 1-10 x 10⁸. The preferred ranges are 0.01 x 10⁸ to 10¹⁰ mature T lymphocytes, such as 0.1 x 10⁸ to 10¹⁰, 1 x 10⁸ to 10¹⁰ or 1 x 10⁹ to 10¹⁰ mature T lymphocytes.

Thus, the mature T-cell sample acquired for autotransplantation is at least 0.01 x 10⁸, generally in the range of 10⁸-10¹⁰ CD3⁺ mature T-cells, preferably 2 x 10⁸ - 10¹⁰, more preferably 3 x 10⁸ - 10¹⁰ CD3⁺ and even more preferably 4-5 x 10⁸ - 10¹⁰ CD3⁺ mature T-cells.

Conveniently, each sample prepared for autotransplantation contains 3 x 10⁸ CD3⁺ mature T-cells, more preferably 5 x 10⁸ and even more preferably 1x14⁹ CD3⁺ mature T-cells. If sufficient resources of blood are available from an individual, even more preferably still 4-5 x 10⁹ CD3⁺ mature T-cells or 10¹⁰ CD3⁺ mature T-cells may be used.

Preferably, the mature T-cell subpopulation sample acquired for autotransplantation which is CD3⁺ and CD8⁺ is at least 0.01 x 10⁸, generally in the range of 0.25 x 10⁸-0.25 x 10¹⁰, and more preferably 0.5 x 10⁸-0.25 x 10¹⁰, and even more preferably 0.75 x 10⁸ - 0.25 x 10¹⁰, and even more preferably still 0.75 x 10⁸ - 0.25 x 10¹⁰ or 1.00 - 1.25 x 10⁸ - 0.25 x 10¹⁰. Specific CD3⁺ and CD8⁺ cell numbers in each sample prepared for grafting is conveniently of the order of 0.2 x 10⁸, preferably 0.4 x 10⁸, or more preferably 1 x 10⁸, or still more preferably 2 x 10⁸, or more preferably 3 x 10⁸, or more preferably 5 x 10⁸. If sufficient resources from an individual are available, 1 x 10⁹, preferably 2 x 10⁹, 4 x 10⁹, or more preferably 1 x 10¹⁰ CD3⁺ and CD8⁺ cells may be used.

Preferably, the mature T-cell subpopulation sample acquired for autologous transplantation which is CD3⁺ and CD4⁺ is at least 0.01 x 10⁸, generally in the range of 0.1 x 10⁸ - 0.5 x 10¹⁰, and more preferably 0.65 x 10⁸ - 0. 5 x 10¹⁰, and even more preferably 0.85 x 10⁸ - 0.5 x 10¹⁰, and even more preferably still 1 x 10⁸ - 0.5 x 10¹⁰ or 1.8 - 3.6 x 10⁸ - 0.5 x 10¹⁰. Specific CD3⁺ and CD4⁺ cell numbers in each sample prepared for grafting is conveniently of the order of 0.2 x 10¹⁰, preferably 0.3 x 10⁸, or more preferably 0.4 x 10⁸, 0.5x10⁸, 1 x 10⁸, 2 x 10⁸, 3 x 10⁸, 4 x 10⁸, or more preferably 5 x 10⁸. If sufficient resources from an individual are available, I x 10⁹, or more preferably 2 x 10⁹, or more preferably 1 x 10¹⁰ CD3⁺ and CD4⁺ cells may be used.

Preferably, the mature T-cell natural killer subpopulation sample acquired for autotransplantation which is CD3⁺ and CD16/56⁺ is at least 0.01 x 10⁸, generally in the range of 0.01 x 10⁸ - 0.5 x 10¹⁰, preferably 0.02 x 10⁸ - 0.5 x 10¹⁰, more preferably 0.03 x 10⁸ - 0.5 x 10¹⁰, and even more preferably still 0.5 x 10⁸ -0.5 x 10¹⁰ or 0.5-2 x 10⁸ to 0.5 x 10¹⁰. Specific CD³⁺ and CD16/56⁺ cell numbers in each sample prepared for grafting is conveniently of the order of 0.01 x 10⁸, 0.2 x 10⁸, 0.3 x 10⁸, 0.5 x 10⁸, 1 x 10⁸, 2 x 10⁸, 3 x 10⁸, 5 x 10⁸, or more preferably, if sufficient resources are available, I x 10⁹, or more preferably 2 x 10⁹, or more preferably 1 x 10¹⁰ CD3⁺ and CD16/56⁺ cells may be used.

In addition, the mature lymphocyte cell sample may preferably include B cells, such as CD19⁺ B lymphocytes. The mature B-cell sample included in the T-cell sample may be at least 10⁷, 10⁸ or 10⁹, generally in the range of 10⁷ -10¹⁰ mature B-cells and preferably 2 x 10⁷-10¹⁰ mature B-cells, more preferably 3 x 10⁷-10¹⁰ mature B-cells, and even more preferably 4-5 x 10⁷ - 10¹⁰ mature B-cells.

Specific numbers of B-cells in autograft is conveniently of the order of 3 x 10⁷, preferably 5 x 10⁸, more preferably 1 x 10⁸ mature B-cells, and even more preferably still 4-5 x 10⁹ or 10¹⁰ mature B-cells.

In addition, the lymphocyte cell sample may preferably include dendritic cells. The dendritic cell sample may be at least 10⁷, 10⁸ or 10⁹ in number, and generally in the range of 10⁷-10¹⁰ dendritic cells and preferably 2 x 10⁷ -10¹⁰ cells, more preferably 3 x 10⁷ - 10¹⁰ cells, and even more preferably 4-5 x 10⁷ - 10¹⁰ cells.

Specific numbers of dendritic cells in an autograft is conveniently of the order of 3 x 10⁷, preferably 5 x 10⁸, more preferably 1 x 10⁸, and even more preferably still 4-5 x 10⁹ or 10¹⁰ mature B-cells.

### XIII. Equivalents

The foregoing description details presently preferred embodiments of the present invention which are therefore to be considered in all respects as illustrative and not restrictive. Those skilled in the art will recognize, or be able to ascertain, using no more than routine experimentation, many equivalents, modifications and variations to the specific embodiments of the invention described specifically herein. Such equivalents, modifications and variations are intended to be (or are) encompassed in the scope of the following claims.

## Claims

1. A process for producing a leukocyte bank suitable for CAT therapy comprising the steps of:
(a) providing a blood sample from a healthy donor individual;
(b) selectively separating leukocytes from the sample;
(c) collecting the separated leukocytes in a holding vessel;
(d) transferring two or more aliquots of the separated leukocytes to independent double containment storage vessels, wherein each of the storage vessels contains, or is in fluid communication with, a cryopreservation medium;
(e) mixing each aliquot with the cryopreservation medium within each storage vessel;
(f) cryogenically preserving each of the two or more aliquots within each of the storage vessels;
(g) retrievably depositing each of the two or more storage vessels containing the aliquots of preserved leukocytes into two or more independent storage systems to produce a leukocyte bank which exhibits deposit redundancy;
wherein:
(h) steps (b) to (g) are conducted within a closed or functionally closed system and are applied iteratively to a series of blood samples from different healthy donor individuals; and
(i) the process further comprises digitally storing information obtained from each donor individual in a digital information unit so as to permit matching of leukocyte deposit and donor for later autologous transplantation;
and optionally wherein
(a) the cryogenic preservation step (f) comprises freezing to a temperature at or below about -160°C; or
(b) the cryogenic preservation step (f) comprises freezing to a temperature at or below about -269°C.

2. The process of claim 1 wherein the cryopropreservation medium comprises a penetrating cryoprotectant;
for example wherein the penetrating cryoprotectant comprises DMSO (e.g. wherein the DMSO is present at a concentration of up to 10%);
and optionally wherein (a) the cryopreservation medium further comprises an anticoagulant (e.g. wherein the anticoagulant comprises an anticoagulant selected from acid citrate dextrose, EDTA and heparin); and/or (b) the cryopreservation medium further comprises a nuclease (e.g. wherein the nuclease comprises ribonuclease and/or deoxyribonuclease).

3. The process of claim 2 wherein (a) the cryopreservation medium further comprises a physiologically acceptable medium such as phosphate buffered saline; and/or (b) the cryopreservation medium further comprises a proteinaceous and/or sugar and/or polysaecharide composition;
and optionally wherein the proteinaceous composition comprises blood serum or a blood serum component; for example wherein the proteinaceous composition comprises blood albumin (such as bovine serum albumin or human serum albumin); e.g. wherein the proteinaceous composition comprises human blood serum isolated from the blood sample of the donor individual.

4. The process of any one of the preceding claims wherein the healthy donor individual:
(a) is predisposed to a leukocyte deficiency; and/or
(b) is not in remission from a leukocyte deficiency; and/or
(c) is juvenile, adolescent or adult; and/or
(d) is at risk of developing a leukocyte deficiency; and/or
(e) is a human individual between the ages of about 12 to 30 (e.g. 15 to 25); and/or
(f) has a fully-developed immune system.

5. The process of any one of the preceding claims wherein the blood sample is an isolated blood sample (for example wherein the isolated blood sample has a volume of 450 to 500ml).

6. The process of any one of the preceding claims wherein steps (b) to (g) are applied iteratively to a series of blood samples from the same healthy donor individual; for example wherein steps (b) to (g) are applied to 2-12 samples taken from the same healthy donor individual over the course of one year.

7. The process of any one of the preceding claims wherein the leukocytes separated in step (b) comprise (or consist essentially of):
(a) granulocytes; and/or
(b) lymphocytes; and/or
(c) monocytes;
and optionally wherein the separation step (b) comprises the selective separation of a particular class or type of leukocyte (for example wherein the separation step (b) comprises the selective separation of B-cells and/or T-cells and/or dendritic cells and/or mixtures thereof).

8. The process of any one of the preceding claims wherein the information stored in step (i) comprises:
(a) genetic information; and/or
(b) the date at which the blood sample was collected from the donor individual; and/or
(c) the age and sex of the donor individual; and/or
(d) the clinical status of the donor individual; and/or
(e) a medical history of the donor individual; and/or
(f) biographical data identifying the donor individual; and/or
(g) details of the processing and storage conditions used; and/or
(h) data identifying the person(s) responsible for processing the sample(s);
for example wherein the information comprises genetic information selected from:
(a) sequence information relating to one or more gene(s); and/or
(b) single nucleotide polymorphism (SNP) data; and/or
(c) a genetic fingerprint.

9. The process of any one of the preceding claims wherein (a) the digital information unit comprises at least one digital computer; and/or (b) the process further comprises the step of labelling the storage vessels (e.g. by physical attachment of a bar code to the storage vessels) of step (f) with information sufficient to permit matching of the leukocyte deposit and donor.

10. The process of any one of the preceding claims further comprising the step of labelling the storage vessels of step (f) with information;
(f) describing the contents of the vessel (for example, sample size, number and/or volume); and/or
(g) identifying the leukocyte bank; and/or
(h) recording the date at which the blood sample was collected from the donor individual; and/or
(i) comprising a statement that each package is for single patient use only; and/or
(j) comprising instructions for opening, aseptic presentation and further storage.

11. The process of any one of the preceding claims wherein the leukocytes are treated;
(a) *in vivo* prior to provision of the blood sample;
(b) *in vitro* prior to separation step (b);
(c) *in vitro* after separation step (b) but prior to preservation step (f);
(d) *in vitro* after preservation step (f).

12. The process of any one of the preceding claims wherein the leukocytes are selectively separated and or collected from the sample by:
(a) isolated leukapheresis;
(b) continuous or interrupted flow centrifugation leukapheresis (e.g. automated continuous or interrupted flow centrifugation leukapheresis);
(c) continuous or interrupted flow filtration leukapheresis (e.g. automated continuous or interrupted flow filtration leukapheresis);
(d) gravity leukapheresis;
(e) batch centrifugal separation and collection (e.g. by bag pressing).

13. A process for producing a leukocyte composition for autotransplantation into a donor individual comprising the steps of:
(e) producing a leukocyte cell bank by the process of any one of the preceding claims;
(f) matching the donor individual with a leukocyte deposit to identify an autologous leukocyte deposit using the information stored in step (h);
(g) retrieving a storage vessel containing an aliquot of preserved autologous leukocytes; and
(h) revitalizing the preserved autologous leukocytes to produce a leukocyte composition for autotransplantation into the donor individual;
and optionally wherein the leukocytes are revitalized by thawing and/or dilution (for example for producing a leukocyte composition for restorative autotransplantation).

14. A leukocyte bank obtainable (or obtained) by the process of any one of claims 1 to 12.

15. A leukocyte composition obtainable (or obtained) by the process of claim 13; for example for use in therapy or prophylaxis or for the manufacture of a medicament for use in autotransplantation (e.g. in restorative autotransplantation).
